# EUROPÄISCHE PATENTSCHRIFT

(11) **EP 0 324 184 B1**
(45) Veröffentlichungstag und Bekanntmachung des Hinweises auf die Patenterteilung: **28.09.1994**
(21) Anmeldenummer: 88121900.0
(22) Anmeldetag: 30.12.1988
(51) Int. Cl.: C07D 217/08, A61K 31/47

(54) **2-Amino-1,2,3,4-tetrahydroisochinolin-Derivate, Verfahren zu ihrer Herstellung und ihre Verwendung als Arzneimittel**
2-Amino-1,2,3,4-tetrahydroisoquinoline derivatives, method for their preparation and their use as medicines
Dérivés de la 2-amino-1,2,3,4-tétrahydroquinoléine, procédé pour leur préparation et leur utilisation comme médicaments

(30) Priorität: 30.12.1987 HU 613287
(43) Veröffentlichungstag der Anmeldung: 19.07.1989
(73) Patentinhaber: CHINOIN Gyogyszer és Vegyészeti Termékek Gyára RT., H-1045 Budapest IV (HU)
(72) Erfinder: Pálosi, Endre, Dipl.-Ing.Chem., H-1026 Budapest (HU); Korbonits, Dezsó, Dr.Dipl-Ing.Chem., H-1025 Budapest (HU); Molnár, Erzsébet, Dipl.-Ing.Chem., H-2133 Szódliget (HU); Szvoboda, Ida, Dipl.-Ing. Chem., H-2120 Dunakeszi (HU); Hársing, László, Dr., H-1136 Budapest (HU); Simon, György, Dr., H-1056 Budapest (HU); Gergely, Vera, Dr., H-1095 Budapest (HU); Körmöczi, Péter, Dr., H-1014 Budapest (HU); Virág, Sándor, Dr., H-1136 Budapest (HU); Mármarosi, Katalin, Dipl.-Ing. Chem., H-2051 Biatorbágy (HU)
(74) Vertreter: Beetz & Partner Patentanwälte

(56) Entgegenhaltungen:
- US-A- 3 037 984
- ARZNEIMITTEL FORSCHUNG, Band 13, Nr. 4, April 1963, Seiten 269-280, Aulendorf, DE; E. JUCKER et al.: "Konstitution und salidiuretischer Effekt von 3-Sulfamyl-4-chlorbenzoesäure-Derivaten und verwandten Verbindungen"

## Beschreibung

Die Erfindung betrifft neue 2-Amino-1,2,3,4-tetrahydroisochinolin-Derivate, ein Verfahren zu ihrer Herstellung, diese Verbindungen enthaltende pharmazeutische Mittel, deren Herstellung sowie die Verwendung der neuen Verbindungen als Diuretika, Saluretika und/oder Antihypertensiva.

Die neuen Verbindungen entsprechen der allgemeinen Formel I, 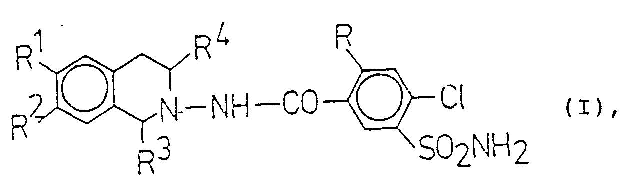
worin bedeuten:
- R: Wasserstoff oder Chlor,
- R¹ und R²: unabhängig Wasserstoff, Methoxy oder Ethoxy

und
- R³ und R⁴: unabhängig Wasserstoff oder Methyl.

Die neuen Verbindungen wirken diuretisch, saluretisch und blutdrucksenkend.

Diuretika vom Typ des Chlorbenzolsulfonamids, die am Benzolring eine freie Carboxylgruppe (Furosemid, vgl. DE 1 122 541 A bzw. K. Sturm, W. Siedel, R. Weyer, H. Ruschig, Chem. Ber. 99, (1966), 328), eine Carbonsäureamidgruppe (Diapamid, vgl. DE 1 158 927 A bzw. L.T. Blouin, D.H. Kaump, R.L. Fransway, D. Williams, J. New Drugs 3, (1963), 302) oder eine Carbonsäurehydrazidgruppe enthalten (Clopamide, vgl. HU-A-150 352 und HU-A-152 300 bzw. A. Lindenmann, E. Schenker, E. Fluckinger, M. Taeschler, Arzneim.-Forschung 13, (1963), 269) sind bereits seit langem bekannt.

Aus der letztgenannten Publikation ist neben dem Clopamide (2,6-Dimethyl-N-[(3'-sulfamoyl-4'-chlorbenzoyl)-amino]-piperidin (DT-327)) (vgl. dort Tabelle 10, Verbindung Nr. 16) die Verbindung N-[(3'-Sulfamoyl-4'-chlorbenzoyl)-amino]-1,2,3,4-tetrahydrochinolin (vgl. dort Tabelle 10, Verbindung Nr. 23) als Salidiuretikum bekannt, wobei die Wirksamkeit von Clopamide im Tierversuch an der Ratte etwa fünfzigmal höher ist als die der Verbindung Nr. 23.

Aus Martindale, The Extra Pharmacopoeia, 28th Edition, The Pharmaceutical Press, London, 1982, Seite 592, war ferner bekannt, daß Clopamide bei längerer Behandlung oder bei empfindlichen Patienten durch vermehrte Kaliumausscheidung zu einer Hypokaliämie führen kann, was eine unerwünschte Nebenwirkung dieses Diuretikums darstellt.

Wie sich aus The Journal of Pharmacology and Experimental Therapeutics 160 (No. 2) (1968), Seite 369, ergibt, beträgt das Na/K-Verhältnis im Tierversuch an Hunden etwa 5.

Die chemische Struktur der erfindungsgemäßen Verbindungen unterscheidet sich von der Struktur der oben erwähnten bekannten Diuretika bedeutend. Verbindungen mit erwähnenswerter diuretischer Wirkung, die ein Isochinolinringsystem enthalten, sind bisher nicht bekannt geworden. Es ist deshalb überraschend, daß die Verbindungen der allgemeinen Formel I eine bedeutende diuretische und saluretische Wirkung aufweisen. In an Ratten vorgenommenen Screening-Untersuchungen (5 mg/kg oral) wurden die erfindungsgemäßen Verbindungen mit Dihydrochlorothiazid und Furosemid verglichen. Hinsichtlich der ausgeschiedenen Mengen an Urin und Alkalimetallionen erwiesen sich die gemäß den Beispielen 1 und 2 hergestellten Verbindungen als besonders vorteilhaft, was insbesondere auch in einem sehr günstigen Na/K-Verhältnis zum Ausdruck kommt.

Besonders vorteilhaft ist, daß die erfindungsgemäßen Verbindungen nicht nur eine ausgezeichnete Wirksamkeit besitzen, sondern ihre therapeutische Anwendung auch vorteilhafter ist als die der "high ceiling" Verbindungen, weil Eintritt und Verlauf von Diurese und Salurese nicht so schnell und heftig erfolgen. Die Wirkung hält ferner nach der Verabreichung etwa 24 Stunden an.

Die akute Toxizität der erfindungsgemäßen Verbindungen ist wesentlich geringer als die der zum Vergleich herangezogenen Verbindungen, und infolgedessen ist ihr therapeutischer Index wesentlich vorteilhafter.

Die erfindungsgemäßen pharmazeutischen Mittel enthalten eine oder mehrere Verbindungen der Formel I als Wirkstoff.

Zur Anwendung in der Humantherapie sind für die orale Verabreichung Tabletten, Dragees oder Kapseln geeignet, die etwa 1-300 mg Wirkstoff und übliche Hilfs- und/oder Trägerstoffe, z.B. übliche Füll- und Streckmittel, enthalten. Die Herstellung der Mittel erfolgt entsprechend durch Formulierung der Wirkstoffe mit üblichen Hilfs- und/oder Trägerstoffen.

Die Verbindungen der allgemeinen Formel I mit R, R¹, R², R³ und R⁴ wie oben werden erfindungsgemäß hergestellt durch Umsetzung von 2-Aminoisochinolin-Derivaten der allgemeinen Formel II 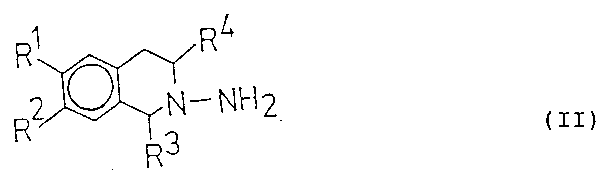
mit R¹, R², R³ und R⁴ wie oben
mit Carbonsäurederivaten der allgemeinen Formel III, 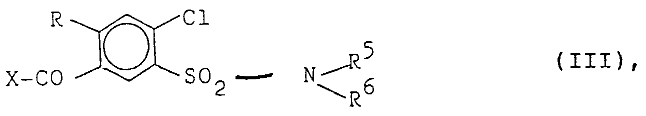
worin bedeuten:
R das gleiche wie oben,
X Chlor, -OH, -OCH₂CN, -OCH₃, -OC₂H₅, -OCOOCH₃ oder -OCOOC₂H₅ und
R⁵ und R⁶ jeweils Wasserstoff oder zusammen die Gruppe =CH-N(CH₃)₂,
und
Abspaltung der Schutzgruppe(n) von den erhaltenen Verbindungen der allgemeinen Formel Ia 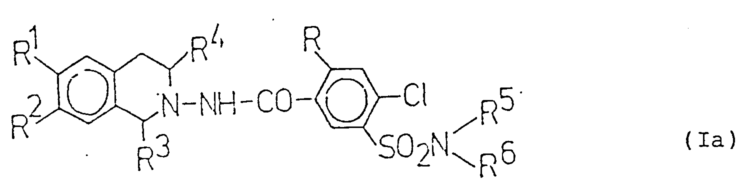
beziehungsweise Ib 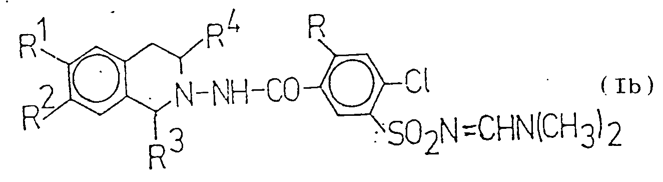
mit R, R¹, R², R³ und R⁴ wie oben
in einem Medium, das eine starke anorganische Base enthält.

Diese Reaktionen entsprechen dem folgenden Reaktionsschema A. 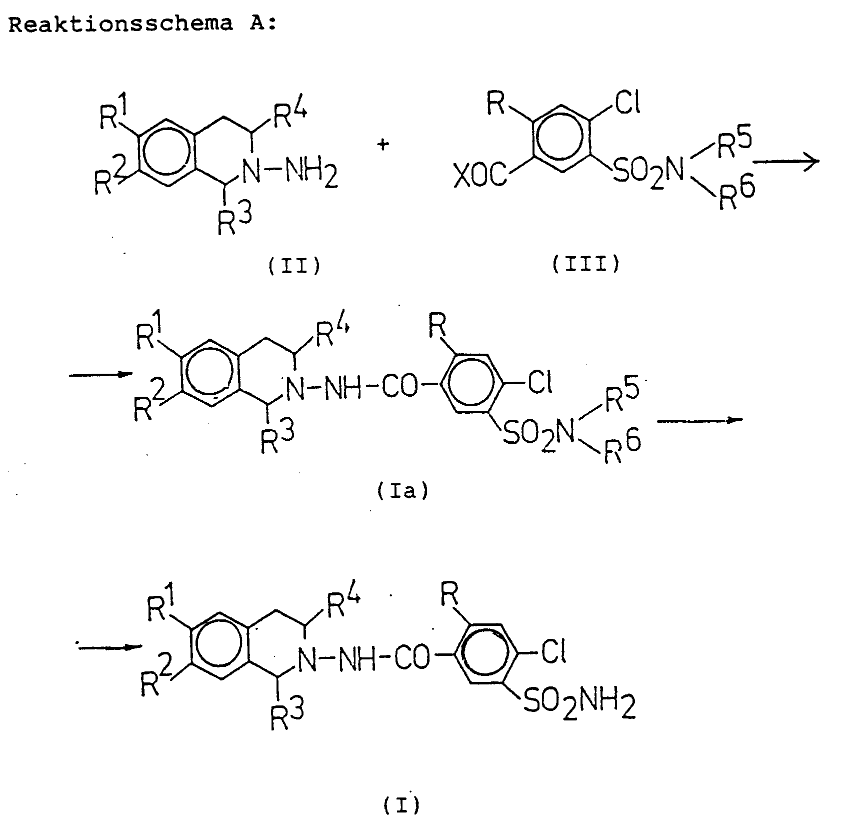
Bei dieser Reaktion können zum Acylieren die freie Carbonsäure oder ein reaktionsfähiges Derivat davon verwendet werden. Als reaktionsfähige Derivate kommen die Säurehalogenide, die mit niederen Alkanolen gebildeten Ester, die aktiven Ester und die gemischten Anhydride in Frage. Als Alkylester verwendet man in erster Linie die Methyl- und Ethylester; ein geeigneter aktiver Ester ist der Cyanomethylester. Die Ausgangsverbindungen werden in etwa äquimolarem Verhältnis eingesetzt. Die Reaktion wird in Anwesenheit von Triethylamin oder Natriumamid vorgenommen.

In gewissen Fällen ist es vorteilhaft, die Sulfonamidgruppe der Verbindungen der Formel III zu substituieren. Für diesen Zweck hat sich die Kondensation mit Formamidacetalen bewährt, bei der Aminomethylidensulfamide entstehen. Diese Reaktion ist besonders dann vorteilhaft, wenn zur Acylierung gemäß Reaktionsschema A Säurechloride verwendet werden. Diese "geschützten" Säurechloride sind nämlich viel stabiler als ihre die freie Sulfonamidgruppe enthaltenden Analoga. Die Umsetzung kann mit Dimethylformamid-dimethylacetal in Dimethylformamid bei Temperaturen von 40-80 °C vorgenommen werden. Sie erfolgt gemäß Reaktionsschema B.

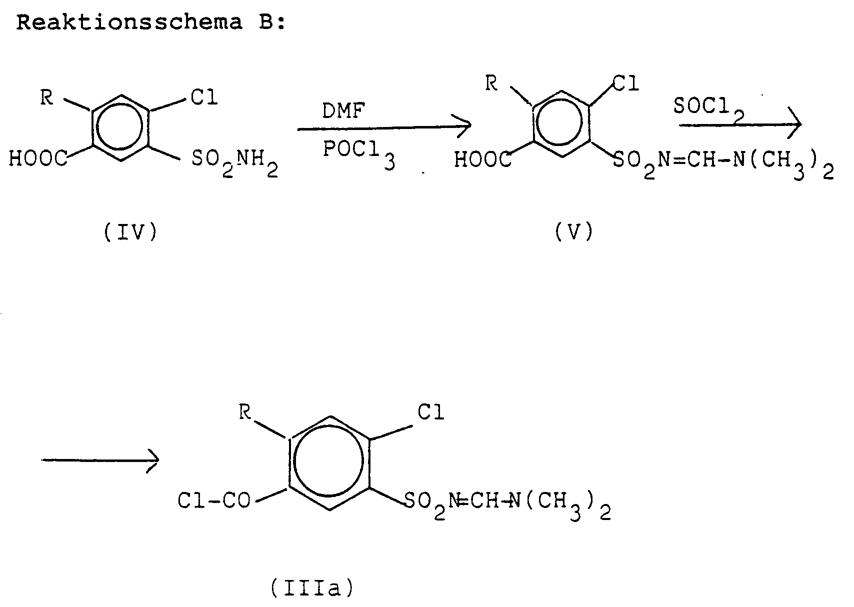

Zur Herstellung der mit der Schutzgruppe versehenen Säure, d.h. der Verbindung V im Reaktionsschema B, ist es besonders vorteilhaft, das Dimethylformamid-dimethylacetal in situ im Reaktionsgemisch herzustellen, das dann sofort mit der Sulfonamidgruppe in Reaktion tritt, wobei die geschützte Säure entsteht. Diese wird dann mit Thionylchlorid in außerordentlich guter Ausbeute zum Säurechlorid der allgemeinen Formel IIIa umgesetzt.

Die Acylierung mit Säurechloriden oder gemischten Anhydriden wird in einem polaren Lösungsmittel vorgenommen. Geeignet sind z.B. Tetrahydrofuran, Dioxan, Pyridin, Dimethylformamid, Dimethylacetamid und Dimethylharnstoff. Die Umsetzung kann bei Temperaturen zwischen -20 °C und dem Siedepunkt des verwendeten Lösungsmittels vorgenommen werden. Wenn das Lösungsmittel nicht basisch ist (wie zum Beispiel Pyridin), so werden als Säurebindemittel organische Basen, wie Triethylamin oder Dimethylanilin, zugesetzt.

Wird mit Säurechloriden der allgemeinen Formel III bzw. IIIa (X = Cl) acyliert, so kann die Umsetzung auch in einem Gemisch aus Wasser und einem mit Wasser mischbaren organischen Lösungsmittel vorgenommen werden, wobei als Säurebindemittel Alkali- oder Erdalkalicarbonate oder -hydrogencarbonate Verwendung finden. Die mit Wasser mischbaren organischen Lösungsmittel können protisch oder aprotisch sein. Als aprotische Lösungsmittel kommen Ether, wie Dioxan und Tetrahydrofuran, Ketone, wie Aceton, oder Säureamide, wie Dimethylformamid und Dimethylacetamid, in Frage, als protische Lösungsmittel sind niedere aliphatische Alkohole, wie Methanol, Ethanol und Propanol, geeignet. Als Alkalicarbonat können Natrium- und Kaliumcarbonat, als Erdalkalicarbonate Magnesium- und Calciumcarbonat und als Alkalihydrogencarbonate Natrium- und Kaliumhydrogencarbonat eingesetzt werden. Die Reaktion wird bevorzugt bei Temperaturen von 0-100 °C und insbesondere von 10-30 °C vorgenommen.

Zur Herstellung des gemischten Anhydrids wird die Säure der allgemeinen Formel V (s. Reaktionsschema B) mit einem Chlorkohlensäurealkylester, insbesondere mit Chlorkohlensäuremethyl- oder -ethylester, umgesetzt. Das gemischte Anhydrid kann isoliert werden; vorteilhafter ist es jedoch, es ohne Isolierung mit der Aminoverbindung der allgemeinen Formel II umzusetzen.

Die Schutzgruppe wird durch basische Hydrolyse entfernt. Die Reaktion erfolgt in wässerigem Medium mit starken anorganischen Basen, vorzugsweise mit Natrium-oder Kaliumhydroxid. Die Reaktionstemperatur liegt bei 20-80 °C und vorzugsweise bei 50-60 °C. Auf 1 mol der zu hydrolysierenden Verbindung werden 2-6 mol und vorzugsweise 3-4 mol der organischen Base eingesetzt.

Wenn die Acylierung mit der freien Carbonsäure der allgemeinen Formel III (X = OH) vorgenommen wird, so ist dem Reaktionsgemisch ein Kondensationsmittel zuzusetzen. Geeignet sind für diesen Zweck zum Beispiel Dicyclohexylcarbodiimid oder Tetrachlorsilan. Als Reaktionsmedium dient zweckmäßig Pyridin.

Aufgrund von an Ratten vorgenommenen Untersuchungen kann festgestellt werden, daß die Verbindungen der Formel I eine ausgezeichnete saluretische Wirksamkeit haben. Die Wirkung tritt 1 bis 2 Stunden nach der Verabreichung ein, erreicht ihr Maximum zwischen der 3. und 5. Stunde und hält 24 Stunden lang an. Dadurch ist eine schonende und langanhaltende Diurese gewährleistet. Besonders vorteilhaft sind das günstige Na/K-Verhältnis und die geringe Toxizität (LD₅₀ > 3000 mg/kg, bestimmt an Mäusen bei oraler Verabreichung).

Die folgenden Beispiele erläutern die Erfindung.

### Beispiel 1

43,3 g 2-Amino-1,2,3,4-tetrahydroisochinolin-hydrochlorid (Herstellung J. Het. Chem. 20, (1983), 121) und 24,5 g Calciumcarbonat werden in 400 ml eines im Verhältnis 2:1 bereiteten Gemisches aus Dioxan und Wasser 30 Minuten lang gerührt. Zu der Suspension werden unter Rühren bei Raumtemperatur in kleinen Portionen 59,5 g 4-Chlor-3-sulfamoylbenzoylchlorid (Herstellung J. Med. Chem. 11, (1968), 970) zugegeben. Nach 2 Stunden wird das Reaktionsgemisch mit 300 ml Wasser verdünnt. Die sich abscheidenden Kristalle werden abgesaugt und mit Wasser, dann mit 0,5 n Salzsäure und anschließend wieder mit Wasser gewaschen. Man erhält 71,9 g (Ausbeute 84 %) 2-[(3′-Sulfamoyl-4′-chlorbenzoyl)-amino]-1,2,3,4-tetrahydroisochinolin in Form weißer Kristalle, das nach basischem Umfällen bei 225-228 °C schmilzt.

| Analyse für C₁₆H₁₆ClN₃O₃S: | | | | | |
|---|---|---|---|---|---|
| berechnet (%): | C 52,53 | H 4,41 | N 11,49 | Cl 9,69 | S 8,77 |
| gefunden (%): | C 52,26 | H 4,41 | N 11,32 | Cl 9,86 | S 8,90. |

### Beispiel 2

Man geht von 28,2 g 1-Methyl-2-amino-1,2,3,4-tetrahydroisochinolin, 17,5 g Calciumcarbonat, 43,2 g 4-Chlor-3-sulfamoylbenzoylchlorid und 350 ml des in Beispiel 1 verwendeten Dioxan-Wasser-Gemisches aus und arbeitet auf die in Beispiel 1 beschriebene Weise. Man erhält 48,3 g (Ausbeute 74,8 %) 1-Methyl-2-[3′-sulfamoyl-4′-chlorbenzoyl)-amino]-1,2,3,4-tetrahydroisochinolin, das sich nach basischem Umfällen bei 227-229 °C zersetzt.

| Analyse für C₁₇H₁₈ClN₃O₃S: | | | | | |
|---|---|---|---|---|---|
| berechnet (%): | C 53,75 | H 4,78 | N 11,06 | Cl 9,33 | S 8,44 |
| gefunden (%): | C 53,86 | H 4,66 | N 11,17 | Cl 9,41 | S 8,48. |

### Herstellung der Ausgangsstoffe

a) Eine Lösung von 21,5 g 1-Methyl-1,2,3,4-tetrahydroisochinolin-hydrochlorid (Herstellung Monatshefte, Band 53/54, (1929), 959) in 50 ml Wasser wird bei 75 °C innerhalb einer Stunde tropfenweise mit einer Lösung von 8,22 g Natriumnitrit in 25 ml Wasser versetzt. Das Reaktionsgemisch wird bei 75 °C 2 Stunden lang gerührt, dann abgekühlt und mit 6 x 30 ml Chloroform extrahiert. Die organische Phase wird mit 50 ml Wasser gewaschen, getrocknet und im Vakuum eingedampft. Man erhält 19,2 g (Ausbeute 93 %) 1-Methyl-2-nitroso-1,2,3,4-tetrahydroisochinolin in Form eines braunen Öls, das ohne weitere Reinigung im folgenden Schritt verwendet wird.
b) Die in Schritt a) erhaltene Nitrosoverbindung wird in 32 ml Eisessig gelöst und unter Eiswasserkühlung und Rühren tropfenweise zu einer Suspension von 31,9 g Zinkstaub in 30 ml Wasser gegeben. Das Reaktionsgemisch wird eine Stunde lang unter Eiswasserkühlung und zwei weitere Stunden lang ohne Kühlung gerührt, dann auf 85 °C erwärmt und filtriert. Das auf dem Filter verbliebene Zink wird mit 3 x 20 ml kochender wässeriger, 5 %iger Salzsäure gewaschen. Das Filtrat wird mit 40 %iger Natronlauge alkalisch gemacht und dann mit 4 x 100 ml Chloroform extrahiert. Die organische Phase wird mit Wasser gewaschen, getrocknet und im Vakuum eingedampft. Man erhält 18 g (Ausbeute 100 %) 1-Methyl-2-amino-1,2,3,4-tetrahydroisochinolin als braunes Öl, das ohne Reinigung weiterverwendet wird.

### Beispiel 3

Man geht von 28,1 g 1-Methyl-2-amino-6,7-dimethoxy-1,2,3,4-tetrahydroisochinolin (Herstellung J. prakt. Chem. 327, (1985), 445), 7 g Calciumcarbonat, 32,1 g 4-Chlor-3-sulfamoylbenzoylchlorid und 20 ml eines 2:1-Gemisches von Dioxan und Wasser aus und arbeitet auf die in Beispiel 1 beschriebene Weise. Man erhält 50 g (Ausbeute 90 %) 1-Methyl-2-[(3′-sulfamoyl-4′-chlorbenzoyl)-amino]-6,7-dimethoxy-1,2,3,4-tetrahydroisochinolin-monohydrat, das bei 230-233 °C schmilzt.

| Analyse für C₁₉H₂₂ClN₃O₅S.H₂O: | | | | | |
|---|---|---|---|---|---|
| berechnet (%): | C 49,83 | H 5,28 | N 9,18 | Cl 7,74 | S 7,00 |
| gefunden (%): | C 49,58 | H 5,00 | N 8,84 | Cl 7,50 | S 6,81. |

### Beispiel 4

Man arbeitet auf die in Beispiel 1 beschriebene Weise mit dem Unterschied, daß die Reaktion in 400 ml eines im Verhältnis 2:1 bereiteten Gemisches aus Isopropanol und Wasser vorgenommen wird. Man erhält 70,1 g (Ausbeute 82 %) 2-[(3′-Sulfamoyl-4′-chlorbenzoyl)-amino]-1,2,3,4-tetrahydroisochinolin, das nach basischem Umfällen bei 226-228 °C schmilzt.

### Beispiel 5

Zu einer Lösung von 22,6 g 2-Amino-1,2,3,4-tetrahydroisochinolin in 400 ml Dioxan wird eine Lösung von 12,7 g wasserfreiem Natriumcarbonat in 200 ml Wasser gegeben. Unter Kühlen mit kaltem Wasser und unter Rühren wird das Reaktionsgemisch tropfenweise mit einer Lösung von 68 g 4-Chlor-3-[(N-dimethylaminomethyliden)-sulfamoyl]-benzoylchlorid in 400 ml Dioxan versetzt, wobei die Temperatur auf 15-20 °C gehalten wird. Nach Beendigung der Zugabe wird das Gemisch bei 20 °C noch 2 Stunden lang gerührt und dann filtriert. Das Filtrat wird mit 2 Liter Wasser verdünnt; die ausgefallenen Kristalle werden abgesaugt, mit Wasser gewaschen und bei 80 °C getrocknet. Das Rohprodukt (63 g) wird in 430 ml 2 n Natronlauge bei 50 °C 8 Stunden lang gerührt. Die Lösung wird mit Aktivkohle geklärt, filtriert; das Filtrat wird unter Kühlen und Rühren mit 2 n Salzsäure auf pH 6 angesäuert. Der ausgefallene Niederschlag wird abgesaugt, mit Wasser gewaschen und bei 80 °C getrocknet. Man erhält 43,9 g (Ausbeute 60 %) 2-[(3′-Sulfamoyl-4′-chlorbenzoyl)-amino]-1,2,3,4-tetrahydroisochinolin, das bei 225-227 °C schmilzt.

### Herstellung des Ausgangsstoffes

Zu einer Suspension von 14,5 g 4-Chlor-3-[(N-dimethylaminomethyliden)-sulfamoyl]-benzoesäure (Herstellung NL-A-76 04356) in 50 ml Thionylchlorid werden zwei Tropfen Dimethylformamid gegeben. Das Reaktionsgemisch wird unter Rühren zwei Stunden lang gekocht und dann filtriert; das Filtrat wird im Vakuum eingedampft. Man erhält 12,7 g (Ausbeute 82 %) 4-Chlor-3-[(N-dimethylaminomethyliden)-sulfamoyl]-benzoylchlorid in Form schneeweißer Kristalle, das bei 140 °C schmilzt. Nach Umkristallisieren aus Benzol liegt der Schmelzpunkt bei 154-155 °C.

| Analyse für C₁₀H₁₀Cl₂N₂O₃S: | | | | | |
|---|---|---|---|---|---|
| berechnet (%): | C 38,84 | H 3,26 | N 9,06 | Cl 22,93 | S 10,37 |
| gefunden (%): | C 38,28 | H 3,07 | N 8,94 | Cl 23,14 | S 10,58. |

### Beispiel 6

Zu einer Suspension von 8,27 g 2-Amino-1,2,3,4-tetrahydroisochinolin in 25 ml Pyridin werden 17,25 g 4-Chlor-3-[(N-dimethylaminomethyliden)-sulfamoyl]-benzoylchlorid gegeben. Das Reaktionsgemisch wird auf etwa 60-70 °C erwärmt, wobei sich eine gelbe Lösung bildet, die man über Nacht stehen läßt. Am anderen Tag wird die Lösung mit 200 ml Wasser versetzt. Dabei scheidet sich ein gelbes Harz ab, das nach einigen Minuten Rühren zu einem sandfarbenen Pulver zerfällt. Das Produkt wird abgesaugt, mit Wasser gewaschen und dann getrocknet. Das Rohprodukt wird auf die in Beispiel 5 beschriebene Weise mit 143 ml 2 n Natronlauge hydrolysiert. Man erhält 15,4 g (Ausbeute 75 %) 2-[(3′-Sulfamoyl-4′-chlorbenzoyl)-amino]-1,2,3,4-tetrahydroisochinolin, das bei 225-227 °C schmilzt.

### Beispiel 7

Man arbeitet auf die in Beispiel 4 beschriebene Weise, nimmt die Umsetzung jedoch in 350 ml eines im Verhältnis 2:1 bereiteten Gemisches aus Ethanol und Wasser vor.

### Untersuchung der saluretischen Wirkung an Ratten

Für die Screening-Untersuchungen wurden männliche Ratten des Stammes LATI CFY mit einer durchschnittlichen Körpermasse von 240 g verwendet. Die Tiere erhielten Standardrattenfutter. 16 Stunden vor Beginn der Versuche wurde die Fütterung eingestellt, die Flüssigkeitsaufnahme jedoch nicht begrenzt. Zum Nachweis der diuretischen Wirkung wurde die von Kagawa und Kalm modifizierte Methode von Lipschitz herangezogen (Arch. Int. Pharmacodyn. 137, (1962), 241-249). Die Tiere wurden in Stoffwechselkäfigen untergebracht. Der Urin wurde von der 0. bis zur 6. Stunde und von der 6. bis zur 24. Stunde gesammelt. Die Kontrolltiere erhielten durch eine Magensonde 25 ml/kg physiologische Kochsalzlösung. Die zu untersuchenden Verbindungen wurden in der gleichen Menge physiologischer Kochsalzlösung und in einer Dosis von 5 mg/kg einmal appliziert. Nach sechs Stunden erhielten die Tiere durch eine Magensonde so viel physiologische Kochsalzlösung, wie die Menge des in den ersten sechs Stunden entleerten Urins betrug. Bestimmt wurden die in der 0. bis 6. Stunde und in der 6. bis 24. Stunde entleerte Urinmenge, die Na- und K-Konzentration des Urins und das Na/K-Verhältnis des in der betreffenden Periode entleerten Urins. Außer der mit physiologischer Kochsalzlösung behandelten Kontrollgruppe wurde auch eine mit Hypothazid behandelte Referenzgruppe mitgeführt.

Die erhaltenen Ergebnisse sind in der nachstehenden Tabelle zusammengefaßt. In der Tabelle sind die Werte der Wasser-, Na- und K-Ausscheidung in Prozent der Werte der unbehandelten Kontrollgruppe angegeben.

| Wirkung erfindungsgemäßer Verbindungen auf die Wasser-, Na- und K-Ausscheidung sowie das Na/K-Verhältnis | | | | | | | | |
|---|---|---|---|---|---|---|---|---|
| Behandlung | Wasserausscheidung | | Na-Ausscheidung | | K-Ausscheidung | | Na/K-Verhältnis | |
| | 0-6 h | 0-24 h | 0-6 h | 0-24 h | 0-6 h | 0-24 h | 0-6 h | 0-24 h |
| Kontrolle | 100 | 100 | 100 | 100 | 100 | 100 | 1,81 | 2,45 |
| Verbindung von Beispiel 1 | 216 | 148 | 260 | 152 | 145 | 82 | 9,09 | 5,94 |
| Verbindung von Beispiel 2 | 233 | 154 | 279 | 151 | 170 | 100 | 7,56 | 5,38 |
| Verbindung von Beispiel 3 | 147 | 112 | 186 | 121 | 104 | 84 | 4,74 | 3,99 |
| Hypothiazid | 159 | 119 | 187 | 123 | 117 | 109 | 3,9 | 3,50 |

## Patentansprüche

1. 2-Amino-1,2,3,4-tetrahydroisochinolin-Derivate der allgemeinen Formel I, 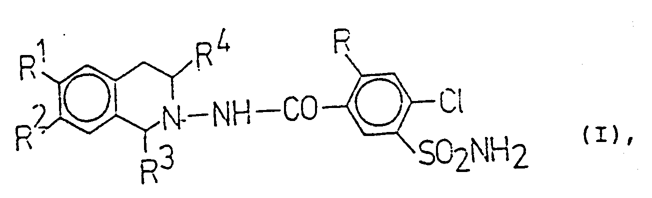 worin bedeuten:
R Wasserstoff oder Chlor,
R¹ und R² Wasserstoff, Methoxy oder Ethoxy und
R³ und R⁴ Wasserstoff oder Methyl.

2. 2-(3'-Sulfamoyl-4'-chlorbenzoyl)-amino-1,2,3,4-tetrahydroisochinolin.

3. 1-Methyl-2-(3'-sulfamoyl-4'-chlorbenzoyl)-amino-1,2,3,4-tetrahydroisochinolin.

4. Verfahren zur Herstellung der 2-Aminoisochinolin-Derivate der allgemeinen Formel I nach einem der Ansprüche 1 bis 3, gekennzeichnet durch Umsetzung von 2-Aminoisochinolin-Derivaten der allgemeinen Formel II 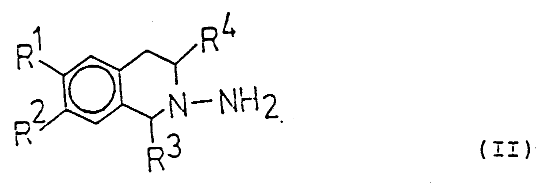 mit R¹, R², R³ und R⁴ wie in Anspruch 1 mit Carbonsäurederivaten der allgemeinen Formel III, 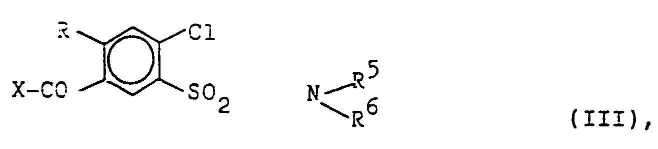 worin bedeuten:
R das gleiche wie in Anspruch 1,
X Chlor, -OH, -OCH₂CN, -OCH₃, -OC₂H₅, -OCOOCH₃ oder -OCOOC₂H₅ und
R⁵ und R⁶ jeweils Wasserstoff oder zusammen die Gruppe =CH-N(CH₃)₂,
und
Abspaltung der Schutzgruppe(n) von den erhaltenen Verbindungen der allgemeinen Formel Ia 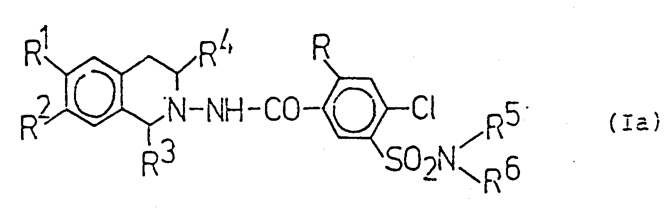 beziehungsweise Ib 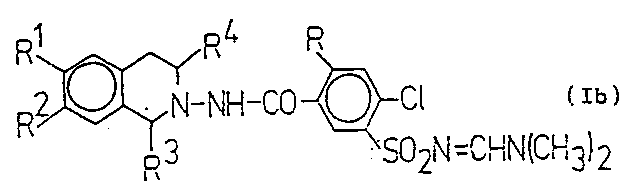 mit R, R¹, R², R³ und R⁴ wie oben
in einem Medium mit einer starken anorganischen Base.

5. Pharmazeutische Mittel, gekennzeichnet durch einen Gehalt an einer oder mehreren Verbindungen der allgemeinen Formel I nach einem der Ansprüche 1 bis 3.

6. Verfahren zur Herstellung der pharmazeutischen Mittel nach Anspruch 5 und insbesondere von diuretisch, saluretisch und/oder antihypertensiv wirkenden pharmazeutischen Mitteln, dadurch gekennzeichnet, daß man Verbindungen der allgemeinen Formel I nach einem der Ansprüche 1 bis 3 zusammen mit üblichen Hilfs- und/oder Trägerstoffen zu entsprechenden pharmazeutischen Mitteln formuliert.

7. Verwendung der Verbindungen der allgemeinen Formel I nach einem der Ansprüche 1 bis 3 zur Herstellung von diuretisch und saluretisch und/oder antihypertensiv wirkenden pharmazeutischen Mitteln.

## Claims

1. 2-Amino-1,2,3,4-tetrahydroisoquinoline derivatives of the general formula I, 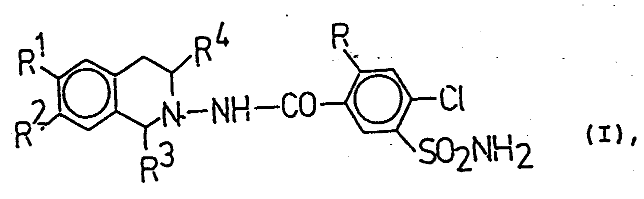 wherein:
R denotes hydrogen or chlorine,
R¹ and R² denote hydrogen, method or ethoxy, and
R³ and R⁴ denote hydrogen or methyl.

2. 2-(3'-Sulphamoyl-4'-chlorobenzoyl)-amino-1,2,3,4-tetrahydroisoquinoline.

3. 1-Methyl-2-(3'-sulphamoyl-4'-chlorobenzoyl)-amino-1,2,3,4-tetrahydroisoquinoline.

4. Process for preparing 2-aminoisoquinoline derivatives of the general formula I according to one of claims 1 to 3, characterised by reacting 2-aminoisoquinoline derivatives of the general formula II 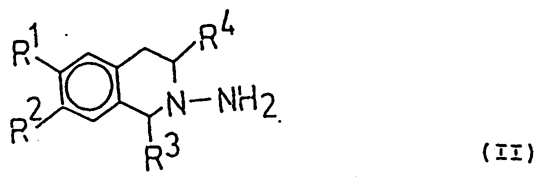 with R¹, R², R³ and R⁴ as in claim 1,
with carboxylic acid derivatives of the general formula III, 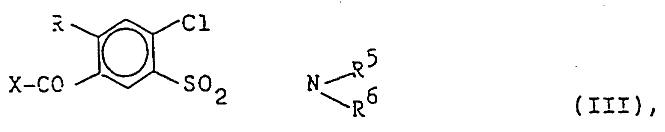 wherein:
R denotes the same as in claim 1,
X denotes chlorine, -OH, -OCH₂CN, -OCH₃, -OC₂H₅, -OCOOCH₃ or -OCOOC₂H₅, and
R⁵ and R⁶ each denote hydrogen or together denote the group =CH-N(CH₃)₂,
and cleaving the protective group(s) from the resulting compounds of the general formula Ia 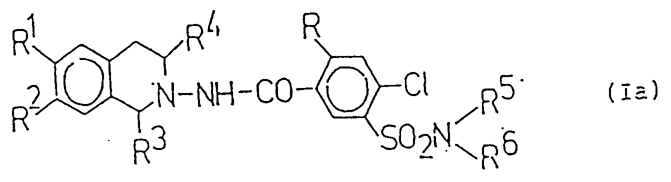 or Ib 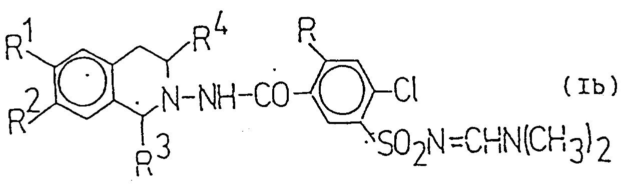 with R, R¹, R², R³ and R⁴ as above,
in a medium using a strong inorganic base.

5. Pharmaceutical agent, characterised by containing one or more compounds of the general formula I according to one of claims 1 to 3.

6. Process for preparing the pharmaceutical agents according to claim 5 and in particular diuretic, saluretic and/or antihypertensive pharmaceutical agents, characterised in that compounds of the general formula I according to one of claims 1 to 3 together with conventional auxiliaries and/or excipients are formulated to produce corresponding pharmaceutical agents.

7. Use of the compounds of the general formula I according to one of claims 1 to 3 for preparing diuretic and saluretic and/or antihypertensive pharmaceutical agents.

## Revendications

1. Dérivés de 2-amino-1,2,3,4-tétrahydroisoquinoléine de formule générale I, 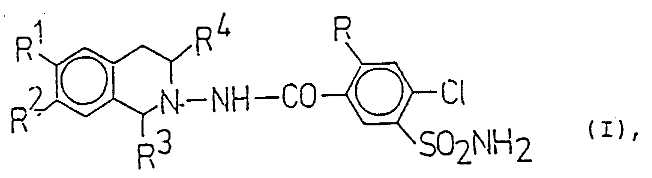 dans laquelle
R représente un atome d'hydrogène ou de chlore,
R¹ et R² représentent un atome d'hydrogène ou le groupe méthoxy ou éthoxy, et
R³ et R⁴ représentent un atome d'hydrogène ou le groupe méthyle.

2. 2-(3'-sulfamoyl-4'-chlorobenzoyl)-amino-1,2,3,4-tétrahydroisoquinoléine.

3. 1-méthyl-2-(3'-sulfamoyl-4'-chlorobenzoyl)-amino-1,2,3,4-tétrahydroisoquinoléine.

4. Procédé pour la préparation des dérivés de 2-amino-isoquinoléine de formule générale I selon l'une des revendications 1 à 3, caractérisé par la réaction de dérivés de 2-aminoisoquinoléine de formule générale II 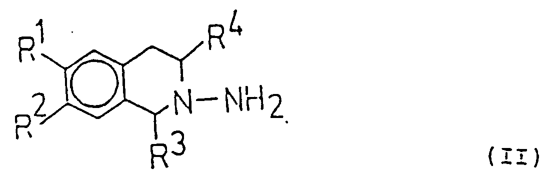 dans laquelle R¹, R², R³ et R⁴ sont tels que dans la revendication 1,
avec des dérivés d'acides carboxyliques de formule générale III, 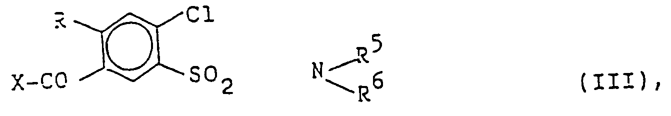 dans laquelle
R a la même signification que dans la revendication 1,
X représente un atome de chlore, -OH, -OCH₂CN, -OCH₃, -OC₂H₅, -OCOOCH₃ ou -OCOOC₂H₅ et
R⁵ et R⁶ représentent chacun un atome d'hydrogène ou, ensemble, le groupe =CH-N(CH₃)₂,
et
l'élimination du(des) groupe(s) protecteur(s) des composés obtenus, de formule générale Ia 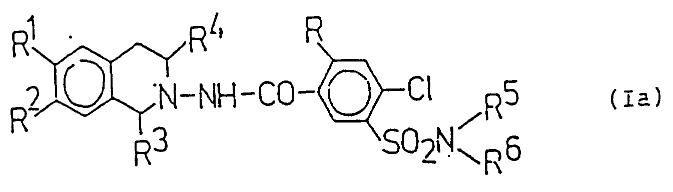 ou Ib 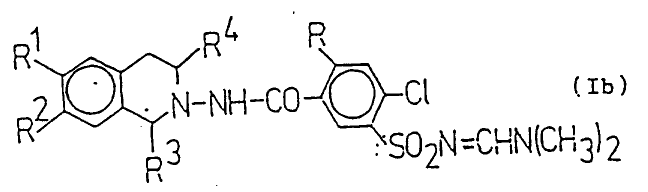 formules dans lesquelles R, R¹, R², R³ et R⁴ sont tels que définis plus haut,
dans un milieu contenant une base minérale forte.

5. Compositions pharmaceutiques caractérisées par une teneur en un ou plusieurs composés de formule générale I selon l'une des revendications 1 à 3.

6. Procédé pour la préparation des compositions pharmaceutiques selon la revendication 5, et en particulier de compositions pharmaceutiques à activité diurétique, salidiurétique et/ou antihypertensive, caractérisé en ce que l'on met sous forme de compositions pharmaceutiques correspondantes des composés de formule générale I selon l'une des revendications 1 à 3, conjointement avec des adjuvants et/ou véhicules usuels.

7. Utilisation des composés de formule générale I selon l'une des revendications 1 à 3, pour la préparation de compositions pharmaceutiques à activité diurétique, salidiurétique et/ou antihypertensive.
